# EUROPEAN PATENT APPLICATION

(11) **EP 1 126 272 A1**
(43) Date of publication of application: **22.08.2001**
(21) Application number: 00870025.4
(22) Date of filing: 18.02.2000
(51) Int. Cl.: G01N 21/86

(54) **Detection and/or quantification device of a precipitate upon the surface of a solid support**

(71) Applicant: Advanced Array Technologies S.A., 5020 Malonne (BE)
(72) Inventor: Demarteau, Joseph, 5000 Namur (BE)
(74) Representative: Van Malderen, Joelle

(57) **Abstract**

The present invention is related to a method for the quantification of a volume of one or more deposit(s) upon a defined surface of a solid support, said solid support having an array of at least 4 discrete regions per cm², each discrete region possibly comprising a deposit, characterised in that images of said defined surface containing one or more deposit(s) and corresponding to different views, said images containing analogue informations, are taken by one or more camera(s) upon illumination by one or more illuminant source(s) spatially arranged relatively to each other according to a predetermined pattern and characterised in the corresponding image analogue informations of said defined surface containing said deposit(s) are transformed and converted into digital form or set of digital forms and compared to a first and to a second reference standards to determine the volume of the deposit(s) to be quantified. The present invention is also related to a device for performing said method and to the use of said method and/or said device for the quantification of one or more target compounds present in a biological sample.

## Description

### Field of the invention

The present invention is related to a method for the quantification of a volume of one or more deposit(s) upon a defined surface of a solid support having microarrays.

The present invention is also related to a device for performing said method.

The present invention is also related to the use of said method and/or said device in the quantification of one or more target compounds present , possibly simultaneously, in a biological sample.

### Background of the invention and state of the art

Biological assays are mainly based upon interaction specificity between two or more biological molecules such as the binding of two strands of nucleic acid molecules, the binding between an antigen and an antibody or the binding between a ligand and its receptor. The present challenge of biological assays is to perform simultaneously the multiple detection of molecules present in a sample. Miniaturisation and development of arrays upon the surface of "biochips" are tools that allow multiplex reactions in a microscopic format, said detection being made with a limited volume of sample for the screening and/or the identification of multiple possible target compounds. These arrays are formed of discrete regions, each one containing a specific capture molecule used for the binding of a corresponding target compound. These discrete regions, as small as a few micrometers or lower, allow the fixation of several thousands or more capture molecules per cm² surface.

However, the detection of bounded target compounds is difficult, since their amount is very small due to said miniaturisation. Typically, the amount of bounded target compounds present in a sample to be detected is usually few fentomoles or even few attomoles. Therefore, only extremely sensitive methods are adequate for such detection.

Sensitive methods to detect and/or quantify the bounded target compounds upon "hybridization chips" have been proposed. Nevertheless, some of these methods are very expensive. That is the case for methods using fluorescent labelled targets and requiring expensive scanners which comprise a laser scanner for excitation of the fluorescent molecules, a pinhole for decreasing the noise fluorescent background, and a photomultiplier for increasing the sensitivity of the detection. The documents US-5,821,060 and WO95/04160 describe other methods using expensive devices such as mass spectrometers.

Methods based upon the precipitation of specific products resulting from enzymatic activities (WO90/06372, EP-A-0444649, EP-A-0124124) have been proposed.

The European patent application 99870106.4 discloses a new method for the detection and/or quantification of multiple target compounds obtained from a biological sample upon biochips showing a high sensitivity. In this method, the target compound is detected and quantified by its binding upon a corresponding capture molecule bound to the surface of a solid support according to an array comprising a density of at least 20 discrete regions per cm2 (each of said discrete regions being bound to one species of capture molecules), the binding between said capture and said target compounds resulting in the formation of a metal precipitate, preferably a silver (Ag) precipitate comprising silver (Ag) crystals, at the location of said capture molecule.

Moreover, this document is also related to a diagnostic/quantification apparatus of multiple target sequences for performing the above-mentioned method. This apparatus comprises: (i) a detection device which can be a CCD or photodiodes to detect the presence of precipitates (spots); (ii) possibly a reading device of information(s) recorded upon said solid support (such as barcodes); (iii) a computer program to possibly recognise the discrete regions bearing capture molecules, to collect the results obtained from said detection device, and possibly correlated with information(s) obtained from the reading device and to carry out a diagnostic and/or quantification of the target compound(s).

Hence, detection resolution, and more particularly the reliability of the final quantification depends largely on the characteristics of the detection device. Especially, when the detection means include a CCD camera, the reliability depends on its number of pixels. The number of pixels thus limits the allowed sensitivity of the quantification. Typically, it is possible to obtain with a CCD a resolution of 10 *µ*m for a pixel, which are sufficient to analyse spots of 100 *µ*m in diameter or bigger. However, such quantification is limited by the number of pixels, by the resolution of each pixel and the fact that the sensitivity is given by only one view point. One view point depends on the three following patterns : the position of the lecture element like CCD camera, the position of the object to be detected and the position of the lightening of the object. Therefore, it exists a need for providing , detection means allowing increased resolution.

### Aims of the invention

The present invention aims to provide a new, simple and not expensive method and device for the quantification of one or more target compounds present (possibly simultaneously) in a biological sample which do not present the drawbacks of the state of the art.

Another aim of the present invention is to provide such a method and device that improve the quantification of said target compounds in terms of sensitivity and speed.

### Summary of the invention

The present invention is related to a method for the quantification of a volume of a deposit preferably containing metal crystals upon a defined surface of a solid support, said solid support having an array of at least 4, at least 10, at least 16, at least 20 or more discrete regions per cm², wherein images of said defined surface comprising one or more deposits corresponding to different views, said images containing analogue informations, are taken by one or several camera(s) and upon illumination by one or several illuminant source(s) being spatially arranged relatively to each other according to a predetermined pattern; the corresponding image analogue informations of said defined surface comprising said deposit(s) are transformed and converted into digital form or set of digital forms and compared to a first and to a second reference standards to determine the volume of the deposit(s) to be quantified.

The first reference standard corresponds to the digital form or set of digital forms obtained from analogue informations contained in images taken on said surface without deposit.

The second reference standard corresponds to the digital form or set of digital forms obtained from analogue informations contained in images taken on said surface comprising deposits of known volume.

The term "volume" should be understood to mean the volume for which it is desired to obtain dimensional-type information. In the present invention, said volume results from a chemical or biochemical reaction following a binding between a target compound and its corresponding compound. Therefore, said obtained volume is the expression of said chemical or biochemical reaction following a binding between a target compound and its corresponding capture compound.

The term "image" should be understood to mean a group of pixels which is an illustration of a measure of said volume and which may be directly transmitted to and registered upon a monitor such as a screen or a printer.

The present invention is also related to a device comprising means for implementing said method, preferably comprising one or several sensor(s) provided with cameras and with one and/or several illuminant source(s) which are spatially arranged relatively to each other according to a predetermined pattern and which are associated with an analogue information acquisition system, the information being measured by using said sensors and being converted into digital form by a processing unit.

Preferably the transformation and conversion are made by a processing unit on board of the camera or in a computer.

Preferably, the cameras are possibly mono, infrared, colour, special adjacent range CCD or CMOS cameras or similar lecture technologies.

Preferably, the illuminant source is an infra-red light having a wavelength similar to the dimension of the metal crystals contained in the deposit(s) and which is advantageously produced by using a single diode or diodes having the same spectral distribution.

Preferably the illuminant sources are regularly spaced around the solid support, each of said sources corresponding to a light spot, which can be automatically switched on simultaneously or successively.

The images are obtained either by transparency, by reflection or by a combination thereof.

As illustrated in the enclosed drawings, the device and method may comprise the use of one camera and one illuminant source, placed above the solid support, said camera and said illuminant source being movable in the three dimensions in space.

The device and method may comprise also the use of two or more cameras oppositely arranged in a plane and placed above the solid support and one or more illuminant sources placed under the solid support.

The device and method may comprise also the use of three or more cameras arranged according to a triangular plane or another regular or irregular pattern and placed above the solid support and one or more illuminant sources placed under the solid support.

The device and method may comprise the use of one camera placed above the solid support, a first illuminant source placed above the solid support and under said camera, a second illuminant source placed under the solid support, the two illuminant sources being placed almost symmetrically according to the position of the solid support.

Alternative preferred embodiments of the present invention are based upon the use of one or more illuminant source(s) and one or more camera(s) which may be used according to the method of the present invention, either in combination or consecutively, said illuminant source(s) and/or said camera(s) can be maintained fixed during the lecture or can be moved according to a preferred translation or rotation movement along or around the solid support comprising the specific volume of a deposit.

It is also possible by using one or more illuminant source(s) and/or one or more camera(s) to allow the movement of the solid support comprising a specific volume of a deposit.

Other embodiments that may be used according to the invention are devices comprising (i) either one camera and several illuminant sources with the different illuminant sources arranged from each other according to different symmetric or non-symmetric patterns, (ii) or one illuminant source and several cameras, said cameras being arranged from each other according to different symmetric or non-symmetric patterns, (iii) or a combination thereof.

The illuminant is an infra-red light having a wavelength similar to the dimension of the crystals contained in the deposit.

Preferably, the deposit upon a defined surface of a solid support is a precipitate comprising silver (Ag) crystals, preferably resulting from the binding between a target chemical or biochemical compound with a corresponding capture chemical or biochemical molecule bound to the surface of said solid support, preferably according to an array comprising a density of at least 4, at least 10, at least 16, at least 20 or more discrete regions per cm², each of said discrete regions being bound to one species of capture molecules, the binding between said capture and said target compounds resulting in the formation of a metal precipitate at the location of said binding according to the method described in the European patent application 99 870 106.4 incorporated herein by reference.

The present invention is finally related to the use of said method and/or said device for the quantification of one or more target compound(s) present in a (preferably biological) sample and preliminary bound to a corresponding capture molecule upon the surface of a solid support according to an array comprising a density of at least 4, at least 10, at least 16, at least 20 or more discrete regions per cm², the binding of said target compound(s) leading to the formation of a precipitate preferably comprising metal crystals.

The target compound(s) to be detected and preferably quantified according to the method of the present invention are any chemical or biochemical compound(s) able to bind to a corresponding capture molecule specific of said target compound(s) according to any physical, chemical or biochemical binding, said binding resulting in the formation of a precipitate at the location of said binding.

The person skilled in the art is able to select the specific target and capture compound(s) able to interact specifically between each other.

Preferably, examples of such biochemical compound(s) able to (bind) interact between each other are for instance biological molecules, such as two strains of nucleic acid sequences, an antigen and an antibody, a ligand and its receptor, possibly present in a biological or a chemical sample to be analysed, can be selected by the person skilled in the art.

The person skilled in the art is also able to provide means for performing the various steps of the present invention, especially the transformation and the conversion of the major volume into digital form or a set of digital forms by using known means or methods such as the ones present in the software and computer technologies.

### Brief description of the drawings

The figure 1 represents the spatial arrangement of some elements in a first embodiment of a device for performing the method according to the invention.

The figure 2 represents the spatial arrangement of some elements in a second preferred embodiment of a device for performing the method according to the invention.

The figure 3 represents the spatial arrangement of some elements in a third preferred embodiment of a device for performing the method according to the invention.

The figure 4 represents the spatial arrangement of some elements in a fourth preferred embodiment of a device for performing the method according to the invention.

The figure 5 represents the spatial arrangement of some elements in a fifth preferred embodiment of a device for performing the method according to the invention.

The figure 6 represents the spatial arrangement of some elements in a sixth preferred embodiment of a device for performing the method according to the invention.

### Detailed description of several preferred embodiments according to the invention

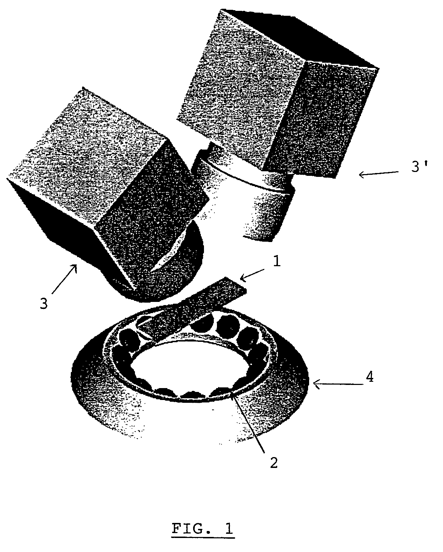

A first preferred embodiment of a device for performing the method according to the invention is shown on figure 1. In this first embodiment, the device comprises a solid support 1, several illuminant sources 2 regularly spaced from each other on a circular support 4, said circular support being placed under said solid support 1, and two cameras 3, 3', said cameras being placed above said solid support 1 and being arranged oppositely in a plane.

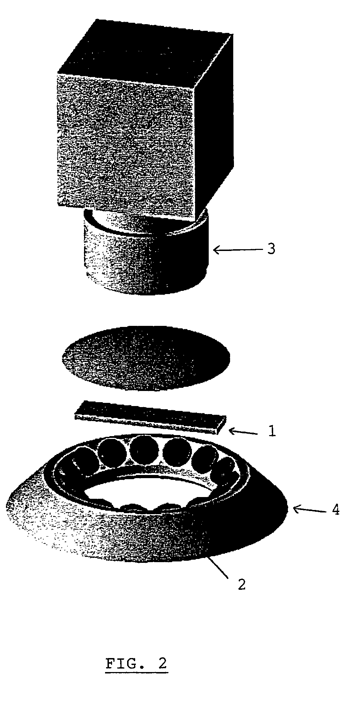

A second preferred embodiment of a device for performing the method according to the invention is shown on figure 2. In this second embodiment, the device comprises a solid support 1, several illuminant sources 2 regularly spaced from each other on a circular support 4, said circular support being placed under said solid support 1, and one camera 3 placed above said solid support 1.

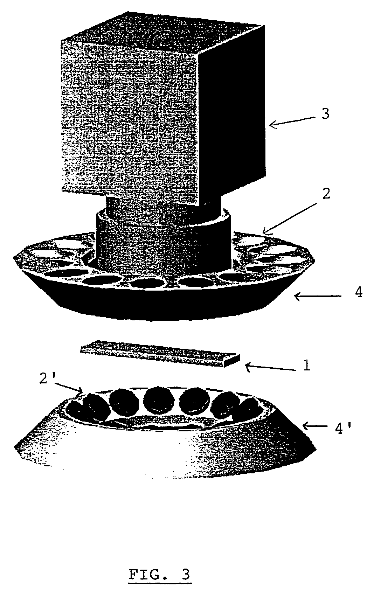

A third preferred embodiment of a device for performing the method according to the invention is shown on figure 3. In this embodiment, the device comprises a solid support 1. The device comprises also a first set of illuminant sources 2 and a second set of illuminant sources 2, the illuminant sources of each set 2, 2' being regularly spaced from each other in a plane, preferably on a circular support 4, 4'. The first set of illuminant sources 2 is placed above the solid support 1 and the second set 2' is placed under said solid support 1, said first and said second sets of illuminant sources 2, 2' being placed symmetrically according to the position of said solid support 1. The device also comprises a camera 3 placed above said solid support 1 and above the first set of illuminant sources 2.

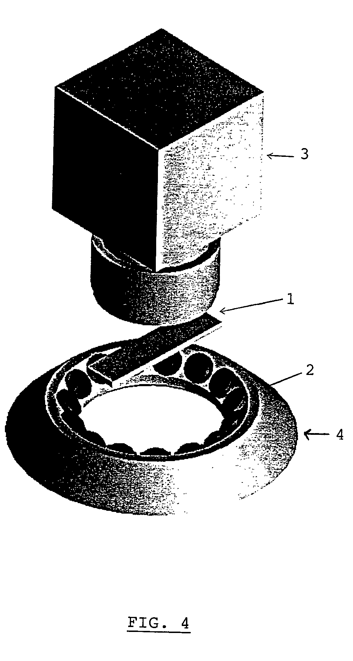

A fourth preferred embodiment of a device for performing the method according to the invention is shown on figure 4. In this embodiment, the device comprises a solid support 1. The device comprises also a several illuminant sources 2 being regularly spaced from each other in a plane, preferably on a circular support 4, and being placed under the solid support 1. The device comprises also a camera 3 placed above. Said circular support 4 and said camera 3 are placed symmetrically according to the position of the solid support 1.

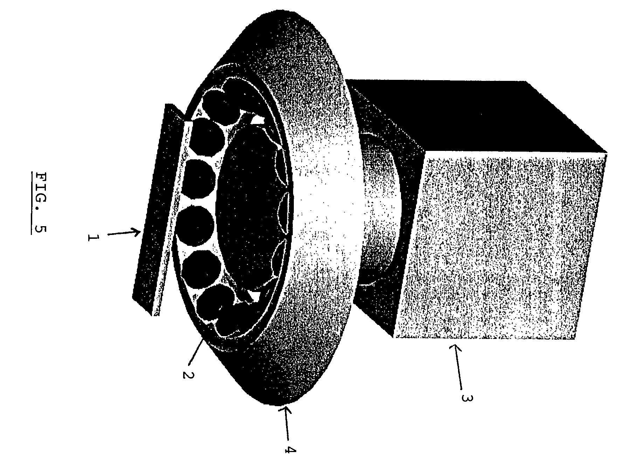

A fifth preferred embodiment of a device for performing the method according to the invention is shown on figure 5. It is a variation of the third embodiment without the second set of illuminant sources 2'.

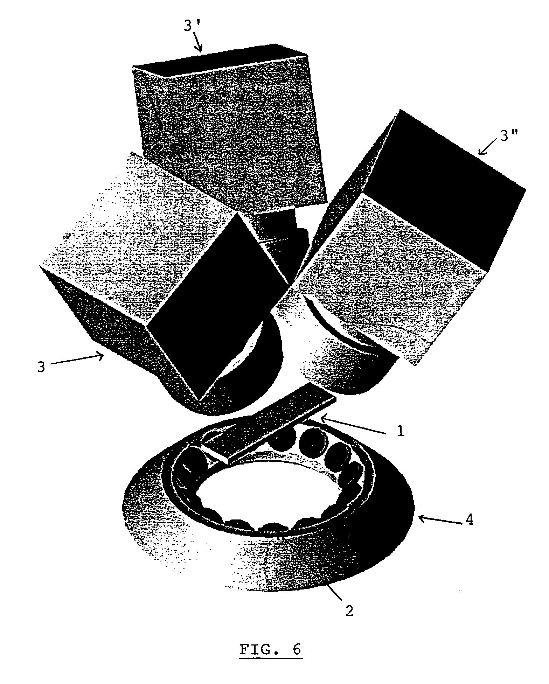

A sixth preferred embodiment of a device for performing the method according to the invention is shown on figure 6. In this sixth embodiment, the device comprises a solid support 1, several illuminant sources 2 regularly spaced from each other in a plane, preferably on a circular support 4, said circular support being placed under said solid support 1, and three cameras 3, 3', 3' ', said cameras being placed above said solid support 1 and being arranged according to triangular arrangement in a plane.

## Claims

1. Method for the quantification of a volume of one or more deposit(s) upon a defined surface of a solid support (1), said solid support (1) having an array of at least 4 discrete regions per cm², each discrete region possibly comprising a deposit, characterised in that images of said defined surface containing one or more deposit(s) and corresponding to different views, said images containing analogue informations, are taken by one or more camera (s) (3, 3', 3' ') upon illumination by one or more illuminant source(s) (2, 2') spatially arranged relatively to each other according to a predetermined pattern and characterised in that the corresponding image analogue informations of said defined surface containing said deposit(s) are transformed and converted into digital form or set of digital forms and compared to a first and to a second reference standards to determine the volume of the deposit(s) to be quantified.

2. Method according to the claim 1, characterised in that the deposit(s) contain metal crystals.

3. Method according to the claim 1 or 2, characterised in that the first reference standard corresponds to a digital form or a set of digital forms obtained from analogue informations contained in images taken on the surface of said solid support (1) without deposit.

4. Method according to the claim 3, characterised in that the second reference standard corresponds to a digital form or a set of digital forms obtained from analogue informations contained in images taken on the surface of said solid support (1) containing deposit(s) of known volume.

5. Method according to any one of the preceding claims, characterized in that each deposit results from the specific binding of a target compound, which has to be detected, to a corresponding capture compound, preferably said capture compound having been fixed on said solid support (1) according to said array before said binding.

6. Device comprising or implementing the method according to any of the preceding claims 1 to 5, comprising one or more sensor(s) provided with camera(s) (3, 3', 3' ') and with one or more illuminant source(s) (2, 2') which are spatially arranged relatively to each other according to a predetermined pattern and which are associated with an analogue information acquisition system, said information being measured by using sensor(s) and being converted into digital form by a processing unit.

7. Device according to claim 5, characterised in that the camera(s) are CCD or CMOS camera(s) (3, 3', 3").

8. Device according to claim 5 or 6, characterised in that the illuminant source (2, 2') is an infra-red light having a wavelength similar to a metal crystal contained in the deposit(s).

9. Device according to any of the preceding claims 5 to 7, characterised in that it comprises a set of illuminant sources (2, 2') regularly spaced from each other in a plane, each of said sources corresponding to a light spot being automatically switched on, simultaneously or successively.

10. Device according to any of the preceding claims 5 to 8, characterised in that it comprises one camera (3) and one illuminant source (2) placed above the solid support, said camera and illuminant source being movable in three dimensions in space.

11. Device according to any of the preceding claims 5 to 8, characterised in that it comprises two or more cameras (3, 3') oppositely arranged in a plane and placed above the solid support, the device comprising further one or more illuminant source (s) (2, 2') placed under the solid support (1).

12. Device according to any of the preceding claims 5 to 8, characterised in that it comprises three or more cameras (3, 3', 3' ') arranged according to a triangular plane or another regular or irregular pattern and placed above the solid support (1) and further comprising one or more illuminant source(s) (2, 2') placed under the solid support (1).

13. Device according to any of the preceding claims 5 to 8, characterised in that it comprises, placed above the solid support (1), one camera (3) and a first illuminant source (2) and, under said camera (3), a second illuminant source (2') placed under the solid support (1), the two illuminant sources (2, 2') being placed almost symmetrically according to the position of the solid support (1).

14. Use of the method according to claim 5 and/or the device according to any one of the claims 6 to 13 for the quantification of one or more target compounds present, possibly simultaneously, in a biological sample.
